# EUROPÄISCHE PATENTANMELDUNG

(11) **EP 3 482 782 A1**
(43) Veröffentlichungstag der Anmeldung: **15.05.2019**
(21) Anmeldenummer: 17201540.6
(22) Anmeldetag: 14.11.2017
(51) Int. Cl.: A61M 1/00

(54) **MEDIZINISCHE SAUGPUMPE**

(71) Anmelder: Medela Holding AG, 6340 Baar (CH)
(72) Erfinder: KÄPPELI, Daniela, 6300 Zug (CH); MARBET, Regina, 4933 Rütschelen (CH); MUTHER, Marcel, 6030 Ebikon (CH); WALTER, Andreas, 8610 Uster (CH)
(74) Vertreter: Clerc, Natalia

(57) **Zusammenfassung**

Eine motorbetriebene medizinische Saugpumpe weist ein Pumpaggregat zur Erzeugung eines Unterdrucks, einen Pumpaggregatträger (3) zur Halterung des Pumpaggregats (7) und eine Aufnahme (35) zur Aufnahme eines Energiespeichers (7) auf. Das im Pumpaggregatträger (3) angeordnete Pumpaggregat (7) definiert eine erste Längsachse (L₁) und die Aufnahme (35) definiert eine zweite Längsachse (L₂). Der Pumpaggregatträger (3) bildet die Aufnahme (35). Die zweite Längsachse (L₂) verläuft in einem Winkel zur ersten Längsachse (L₁). Diese Saugpumpe ist klein und kompakt und weist eine gute Schalldämpfung auf.

## Beschreibung

### TECHNISCHES GEBIET

Die vorliegende Erfindung betrifft eine medizinische Saugpumpe, insbesondere eine motorbetriebene medizinische Saugpumpe. Derartige Saugpumpen sind insbesondere Brustpumpen zum Abpumpen von menschlicher Muttermilch oder Drainagepumpen zum Absaugen von Körperflüssigkeiten, wie beispielsweise für die Thoraxdrainage oder für die Wunddrainage.

### STAND DER TECHNIK

Medizinische Saugpumpen, auch Vakuumpumpen genannt, sind für die verschiedensten Anwendungen bekannt. Beispielsweise werden sie als Brustpumpen zum Abpumpen von menschlicher Muttermilch oder als Drainagepumpen zum Absaugen von Körperflüssigkeiten verwendet. Derartige Saugpumpen weisen als Pumpaggregat Kolbenpumpen oder Membranpumpen auf. Die Verwendung einer Pumpmembran hat den Vorteil, dass die Saugpumpe als Ganzes relativ klein und leicht und im Gebrauch tragbar ausgebildet werden kann. Ein sehr kleines und trotzdem die hohen Anforderungen an eine Brustpumpe erfüllendes motorbetriebenes Pumpaggregat ist aus WO 2006/032156 A1 bekannt. Tragbare Saugpumpen weisen üblicherweise auch einen Energiespeicher zum Betreiben eines Motors des Pumpaggregats auf. Die Grösse des Energiespeichers und des Pumpaggregat bestimmen somit im Wesentlichen die Grösse des Pumpengehäuses.

Eine weitere Anforderung an derartige Saugpumpen ist die Schalldämpfung. Das Pumpaggregat ist oft relativ laut und rhythmisch wiederkehrende Geräusche stören den Benützer. Es ist deshalb bekannt, das Pumpaggregat in eine schalldämmende bzw. schalldämpfende Umgebung einzubetten. In diesem Text wird Schalldämmung und Schalldämpfung gleichwertig behandelt und jeweils als Schalldämpfung bezeichnet.

WO 2017/157691 A1 offenbart eine medizinische Saugpumpe mit einer elastischen Lagerung des Pumpaggregats innerhalb eines Pumpengehäuses.

In WO 2015/109934 A1 ist das Pumpaggregat in einem geschlossenen inneren Gehäuse angeordnet und eine Batterie ist parallel zum inneren Gehäuse in einem äusseren Pumpengehäuse angeordnet.

In WO 2017/140562 A1 ist das Pumpaggregat mittels Federn in einem äusseren Pumpengehäuse befestigt.

### DARSTELLUNG DER ERFINDUNG

Es ist deshalb eine Aufgabe der Erfindung, eine medizinische Saugpumpe zu schaffen, welche möglichst kompakt, jedoch robust ausgebildet ist.

Diese Aufgabe löst eine motorbetriebene medizinische Saugpumpe mit den Merkmalen des Patentanspruchs 1.

Die erfindungsgemässe motorbetriebene medizinische Saugpumpe weist ein Pumpaggregat zur Erzeugung eines Unterdrucks, einen Pumpaggregatträger zur Halterung des Pumpaggregats und eine Aufnahme zur Aufnahme eines Energiespeichers auf, wobei das im Pumpaggregatträger angeordnete Pumpaggregat eine erste Längsachse definiert und die Aufnahme eine zweite Längsachse definiert. Erfindungsgemäss bildet der Pumpaggregatträger die Aufnahme und die zweite Längsachse verläuft in einem Winkel zur ersten Längsachse.

Da der Pumpaggregatträger die Aufnahme für den Energiespeicher bildet und dank der winkligen Anordnung von Pumpaggregat und Energiespeicher sind praktisch keine Leerräume vorhanden und die Grösse der Saugpumpe insgesamt ist minimiert. Da Pumpaggregat und Energiespeicher von demselben Pumpaggregatträger gehalten sind, ist die Vorrichtung insgesamt relativ steif ausgebildet. Dies erhöht nicht nur die Robustheit der Vorrichtung sondern dies minimiert auch Schwingungen beim Betrieb des Pumpaggregats und reduziert somit die Geräuschbildung bzw. -weiterleitung.

In bevorzugten Ausführungsformen ist der Pumpaggregatträger im Wesentlichen als Rahmen ausgebildet. Der Rahmen kann eine beliebige Form aufweisen. Vorzugsweise weist er jedoch eine im Wesentlichen längliche abgerundete Grundform, insbesondere eine elliptische oder ovale Grundform, auf. Die Ausbildung als Rahmen ermöglicht eine Leichtbauweise und erhöht zudem die Steifigkeit. Zudem ist ein Minimum an Material notwendig, was die Herstellungskosten entsprechend minimiert. Der Rahmen ist vorzugsweise einstückig ausgebildet. Vorzugsweise ist er im Spritzgussverfahren aus Kunststoff hergestellt.

Vorzugsweise umfasst die Saugpumpe ein erstes Pumpgengehäuseteil und ein zweites Pumpengehäuseteil, wobei der Pumpaggregatträger zwischen dem ersten und dem zweiten Pumpengehäuseteil gehalten ist. Dadurch wird er in seiner Lage fixiert und seine Steifigkeit wird erhöht.

In bevorzugten Ausführungsformen bildet der Pumpaggregatträger einen Teil des Pumpengehäuses. Er bildet vorzugsweise ein von aussen sichtbares Verbindungsteil zwischen dem ersten und dem zweiten Pumpengehäuseteil. Dies erleichtert den Zusammenbau der einzelnen Bauteile und eine weitere Lagerung des Pumpaggregatträgers innerhalb des Pumpengehäuses lässt sich dadurch vermeiden.

Vorzugsweise ist der gesamte Pumpaggregatträger einstückig ausgebildet.

In bevorzugten Ausführungsformen sind mindestens zwei elastische Lager zur Lagerung des Pumpaggregats im Pumpaggregatträger vorhanden. Dadurch lässt sich die Übertragung von Vibrationen und Körperschall vom Pumpaggregat auf den Pumpaggregatträger und somit auf das Pumpengehäuse minimieren bzw. vermeiden. Zudem ist das Pumpaggregat dadurch definiert gehalten und trotzdem flexibel aufgehängt, so dass die Geräuschentwicklung reduziert und/oder gedämpft wird. Vorzugsweise ist eine Zweipunktlagerung vorhanden. Es ist jedoch auch möglich, eine Drei- oder Mehrpunktlagerung einzusetzen. Die Lager sind vorzugsweise aus einem Elastomer gebildet.

Vorzugsweise bildet mindestens ein erstes dieser Lager einen Vakuumanschluss. Mindestens ein zweites dieser Lager, welches vorzugsweise das Gegenlager zum ersten Lager bildet, ist vorzugsweise an einem motorseitigen Ende des Pumpaggregats und in Verlängerung einer Motorachse des Pumpaggregats, d.h. in der Flucht der Motorachse, angeordnet.

Vorzugsweise sind genau zwei Lager vorhanden, welche entlang der ersten Längsachse an gegenüberliegenden Enden des Pumpaggregats angeordnet sind. Vorzugsweise sind diese zwei Lager bezüglich der ersten Längsachse versetzt zueinander angeordnet. Die Verwendung von genau zwei elastischen Lagern und/oder die genannte Anordnung der Lager minimiert die Grösse der Saugpumpe insgesamt und führt zudem dazu, dass sich Vibrationen und Körperschall nicht oder nur begrenzt ausbreiten können.

Die Schalldämpfung ist optimiert, wenn das Pumpaggregat in einem Schalldämpfungsgehäuse angeordnet ist und das Schalldämpfungsgehäuse im Pumpaggregatträger gehalten ist.

Vorzugsweise ist eine Lufteinlassöffnung vorhanden, welche von einem Schalldämpfungselement überdeckt ist. Vorzugsweise ist die Lufteinlassöffnung im Schalldämpfungsgehäuse angeordnet, welches Zischgeräusche beim Einsaugen der Umgebungsluft minimiert. Das Schalldämpfungselement besteht vorzugsweise aus Schaumstoff oder aus einem anderen geeigneten porösen oder luftdurchlässigen Material. Das Pumpengehäuse ist vorzugsweise auf bekannte Art und Weise undicht ausgebildet, so dass über mehrere Stellen ein Luftaustausch vom Innenraum des Pumpgengehäuses in die Umgebung und umgekehrt stattfinden kann.

Vorzugsweise weist das Schalldämpfungsgehäuse ein Etui mit einer Aufnahmeöffnung zur Aufnahme des Pumpaggregats und einen das Etui verschliessenden Deckel auf, wobei der Deckel gebogen ausgebildet ist. Dies erleichtert den Zusammenbau der Vorrichtung und ermöglicht eine möglichst platzsparende Ausbildung des Schalldämpfungsgehäuses. Das Etui ist vorzugsweise aus einem steifen oder halbsteifen Material, insbesondere aus einem Kunststoff, und der Deckel ist vorzugsweise aus einem weichen Material gefertigt.

Vorzugsweise ist das Schalldämpfungsgehäuse mittels der mindestens zwei elastischen Lager im Pumpaggregatträger gelagert. Vorzugsweise lagern die mindestens zwei elastischen Lager einerseits das Schalldämpfungsgehäuse im Pumpengehäuse und andererseits das Pumpaggregat innerhalb des Schalldämpfungsgehäuses. D.h. dieselben Lager weisen Doppelfunktionen auf, vorzugsweise weist jedes Lager auf zwei gegenüberliegenden Seiten entsprechende Flächen oder Ausbildungen auf. Dadurch reduziert sich die Anzahl der benötigten Lager. Die Lagerung ist genau definiert und optimiert. Zudem benötigt sie wenig Platz und ein Minimum an Bauteilen. Die Schalldämpfung ist verbessert, der Zusammenbau erleichtert und die Herstellungskosten minimiert.

Vorzugsweise ist das erste Lager ein Lagermodul in Form eines elastischen Einsatzelements, welches im Schalldämpfungsgehäuse gehalten ist und dieses durchsetzt. Das gesamte Einsatzelement ist vorzugsweise weich und einstückig hergestellt, beispielsweise aus Silikon oder TPE (thermoplastisches Elastomer). Das Einsatzelement dient als zweiseitiges Lager und verschliesst zudem das Schalldämpfungsgehäuse.

Vorzugsweise ist das zweite Lager ein Deckel oder ein Lagermodul in Form eines Teils eines Deckels des Schalldämpfungsgehäuses, wobei der gesamte Deckel elastisch ausgebildet ist. Vorzugsweise besteht er aus einem weichen Material, wie z.B. Silikon oder TPE. Durch Wahl der Form des Deckels lässt sich die Steifigkeit der Lagerstelle definieren. Der Deckel an sich kann das zweite Lager bilden oder zumindest Teile dieses Lagers bilden.

Einzelne Elemente lassen sich auch ohne die winklige Anordnung von Pumpaggregat und Energiespeicher bzw. ohne die Anordnung dieser zwei Bauteile auf einem gemeinsamen Pumpaggregatträger zu anderen erfindungsgemässen Saugpumpen zusammensetzen. Beispiele hierfür sind nachfolgend angegeben, welche als separate Erfindungen ebenfalls beansprucht sind. Die oben genannten bevorzugten Ausführungsformen, insbesondere die Merkmale der abhängigen Patentansprüche, lassen sich auch entsprechend mit diesen Beispielen ohne Verwendung aller Merkmale des Patentanspruchs 1 kombinieren.

In einer bevorzugten, ebenfalls als Erfindung beanspruchten Ausführungsform weist eine medizinische Saugpumpe ein Pumpengehäuse, ein im Pumpengehäuse angeordnetes Pumpaggregat zur Erzeugung eines Unterdrucks und mindestens ein erstes elastisches Lager und ein zweites elastisches Lager zur Lagerung des Pumpaggregats im Pumpengehäuse auf. Das erste elastische Lager befindet sich an einem ersten Ende des Pumpaggregats und das zweite elastische Lager befindet sich an einem zweiten, dem ersten Ende gegenüberliegenden Ende des Pumpaggregats. Dabei bildet das erste elastische Lager einen Vakuumanschluss. Diese Anordnung ist äusserst platzsparend und vermindert in optimaler Weise Vibrationen.

In einer anderen, ebenfalls als Erfindung beanspruchten Ausführungsform weist eine medizinische Saugpumpe ein Pumpaggregat, ein Schalldämpfungsgehäuse zur Aufnahme des Pumpaggregats und ein Pumpengehäuse zur Aufnahme des Schalldämpfungsgehäuses auf. Das Schalldämpfungsgehäuse weist ein Etui zur Aufnahme des Pumpaggregats und einen das Etui verschliessenden Deckel auf. Der Deckel ist elastisch ausgebildet und er weist ein elastisches Lager zur Lagerung des Schalldämpfungsgehäuses im Pumpengehäuse auf. Diese Ausbildung ermöglicht einen einfachen Zusammenbau der Vorrichtung, da das Pumpaggregat lediglich in das Etui eingeschoben werden muss. Der elastische, insbesondere weiche Deckel verhindert auf optimale Weise Vibrationen und die Verbreitung von Körperschall.

In bevorzugten Ausführungsformen sind diese zwei Varianten miteinander kombiniert. D.h. das erste Lager bildet den Vakuumanschluss und das zweite Lager den Deckel.

In einer weiteren bevorzugten Ausführungsform, welche ebenfalls als separate Erfindung beansprucht wird, weist eine medizinische Saugpumpe ein Pumpaggregat, ein Schalldämpfungsgehäuse zur Aufnahme des Pumpaggregats und ein Pumpengehäuse zur Aufnahme des Schalldämpfungsgehäuses auf. Mindestens ein erstes und ein zweites elastisches Lager sind vorhanden, wobei mindestens eines dieser zwei elastischen Lager einerseits das Schalldämpfungsgehäuse im Pumpengehäuse lagert und andererseits das Pumpaggregat innerhalb des Schalldämpfungsgehäuses lagert. Vorzugsweise lagern beide elastischen Lager sowohl das Schalldämpfungsgehäuse bezüglich des Pumpengehäuses wie auch das Pumpaggregat bezüglich des Schalldämpfungsgehäuses. Das heisst, vorzugsweise sind beide elastischen Lager als Module ausgebildet, welche auf zwei gegenüberliegenden Seiten Lagerstellen bilden. Wie oben bereits erwähnt, ist hier die Lagerung sowie die Schalldämpfung optimiert und eine äusserst kompakte Bauweise ermöglicht. Diese Ausführungsform lässt sich beispielsweise erreichen, indem der Deckel und/oder der Vakuumanschluss als Lager ausgebildet sind und jeweils an den entsprechenden Stellen oder gesamthaft weich bzw. flexibel ausgebildet sind.

Weitere Ausführungsformen sind in den abhängigen Ansprüchen angegeben.

### KURZE BESCHREIBUNG DER ZEICHNUNGEN

Bevorzugte Ausführungsformen der Erfindung werden im Folgenden anhand der Zeichnungen beschrieben, die lediglich zur Erläuterung dienen und nicht einschränkend auszulegen sind. In den Zeichnungen zeigen:
- Figur 1: eine Explosionsdarstellung einer erfindungsgemässen Saugpumpe in einer ersten Ausführungsform;
- Figur 2: einen ersten Querschnitt durch die Saugpumpe gemäss Figur 1;
- Figur 3: einen zweiten Querschnitt durch die Saugpumpe gemäss Figur 1;
- Figur 4: eine Explosionsdarstellung eines Schalldämpfungsgehäuses mit einem Pumpaggregat der Saugpumpe gemäss Figur 1;
- Figur 5: eine perspektivische Darstellung eines Teils der Saugpumpe gemäss Figur 1 ohne Schalldämpfungselement;
- Figur 6: eine perspektivische Darstellung eines Teils der Saugpumpe gemäss Figur 1 mit Schalldämpfungselement;
- Figur 7: eine perspektivische Darstellung eines ersten Lagermoduls der Saugpumpe gemäss Figur 1;
- Figur 8: eine Ansicht des ersten Lagermoduls gemäss Figur 7 von oben;
- Figur 9a: einen Querschnitt durch eine Anschlussöffnung des ersten Lagermoduls gemäss Figur 7 in einer ersten Variante;
- Figur 9b: einen Querschnitt durch eine Anschlussöffnung des ersten Lagermoduls gemäss Figur 7 in einer zweiten Variante;
- Figur 9c: einen Querschnitt durch eine Anschlussöffnung des ersten Lagermoduls gemäss Figur 7 in einer dritten Variante;
- Figur 10: eine perspektivische Darstellung eines zweiten Lagermoduls der Saugpumpe gemäss Figur 1;
- Figur 11: eine Ansicht des zweiten Lagermoduls gemäss Figur 10 von oben;
- Figur 12a: einen Querschnitt durch eine Anschlussöffnung des zweiten Lagermoduls gemäss Figur 11 in einer ersten Variante;
- Figur 12b: einen Querschnitt durch eine Anschlussöffnung des zweiten Lagermoduls gemäss Figur 11 in einer zweiten Variante;
- Figur 12c: einen Querschnitt durch eine Anschlussöffnung des zweiten Lagermoduls gemäss Figur 11 in einer dritten Variante und
- Figur 12d: einen Querschnitt durch eine Anschlussöffnung des zweiten Lagermoduls gemäss Figur 11 in einer vierten Variante.

### BESCHREIBUNG BEVORZUGTER AUSFÜHRUNGSFORMEN

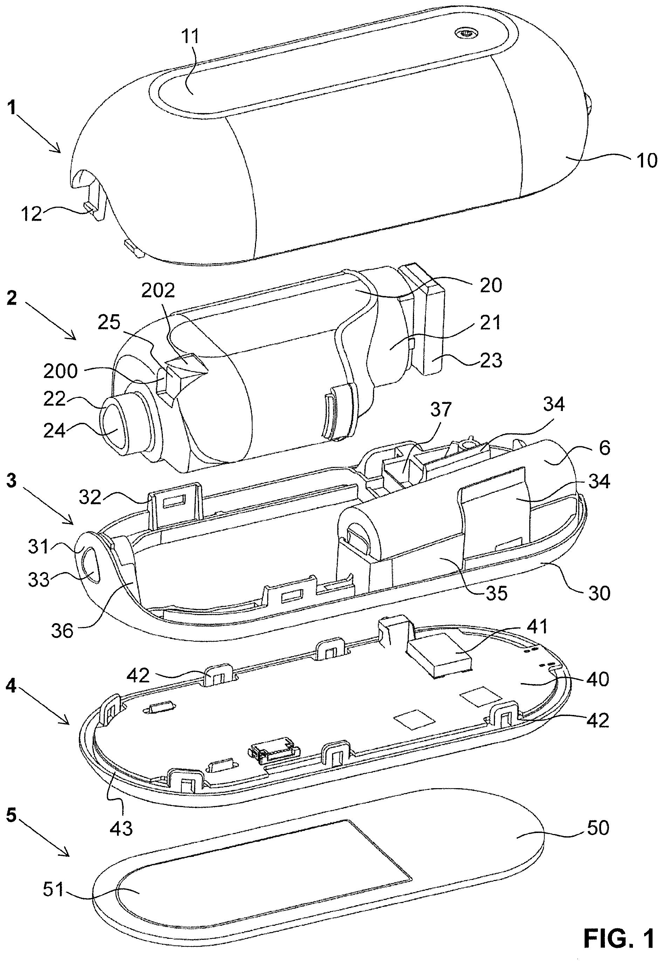

In Figur 1 ist ein bevorzugtes Ausführungsbeispiel einer erfindungsgemässen Saugpumpe dargestellt. Es handelt sich um eine Brustpumpe zum Abpumpen von menschlicher Muttermilch.

Die Saugpumpe weist ein erstes Pumpengehäuseteil 1, ein Schalldämpfungsgehäuse 2, einen Pumpaggregatträger 3, ein zweites Pumpengehäuseteil 4 sowie eine Abdeckung 5 auf. Im zusammengesetzten Zustand der Saugpumpe befindet sich das Schalldämpfungsgehäuse 2 innerhalb eines Pumpengehäuses, welches im Wesentlichen durch das erste Pumpengehäuseteil 1, das zweite Pumpengehäuseteil 4 und den dazwischen angeordneten und zwischen diesen zwei Teilen 1, 4 eingeklemmten Pumpaggregatträger 3 gebildet ist. Der Pumpaggregatträger 3 ist somit vorzugsweise von aussen als umlaufender Streifen erkennbar.

Das erste Pumpengehäuseteil 1 bildet den Boden der Saugpumpe. Es weist einen schalenförmigen Grundkörper 10 auf, dessen Unterseite, welche hier nach oben gerichtet ist, nahezu flach ausgebildet ist und eine Auflagefläche 11 bildet. Im Längsschnitt ist der Grundkörper 10 oval ausgebildet. Am Umfang des Grundkörpers 10 verteilt sind erste Verbindungselemente 12, vorzugsweise Schnappelemente oder andere bekannte Verbindungsmittel, angeordnet.

Das zweite Pumpengehäuseteil 4 weist eine im Wesentlichen ovale Grundplatte 40 und eine umlaufende Rinne 43 zur Aufnahme des Pumpaggregatträgers 3 auf. Auf der Grundplatte 40 sind die in Saugpumpen üblichen elektronischen Bauteile angeordnet. Sie sind hier lediglich schematisch dargestellt. Eine Steuer- und Elektronikeinheit ist mit dem Bezugszeichen 41 versehen. Über dem Umfang des zweiten Pumpengehäuseteils 4 verteilt sind zweite Verbindungselemente 42 angeordnet, die in den Rahmen 30 eingreifen.

Die Abdeckung 5 bildet zusammen mit dem zweiten Pumpengehäuseteil 4 das obere Teil des Pumpengehäuses, auch wenn diese Bauteile in dieser Darstellung unten angeordnet sind. Die Abdeckung 5 lässt sich auf dem zweiten Pumpengehäuseteil 4 befestigen. Sie weist vorzugsweise eine planparallele steife und ovale Deckplatte 50 mit einem Anzeige- und Betätigungsfenster 51 auf. Mittels des Anzeige- und Betätigungsfensters 51 lassen sich beispielsweise Benutzereingaben eingeben und die Funktionsweise der Saugpumpe optisch darstellen.

Der Pumpaggregatträger 3 ist vorzugsweise steif oder halbsteif ausgebildet. Vorzugsweise ist er aus einem Kunststoff gefertigt und vorzugsweise ist er einstückig. Er weist einen ovalen Rahmen 30 auf. An einer schmalen Stirnseite geht der Rahmen 30 in eine erhöhte Stirnwand 31 über, welche eine Durchgangsöffnung 33 aufweist. Die Rückseite der Stirnwand 31 ist mit einer ersten Lageraufnahme 36 versehen, welche die Durchgangsöffnung 33 umgibt. Verteilt über dem Umfang des Rahmens 30 sind dritte Verbindungselemente 32 vorhanden, die in das erste Pumpengehäuseteil 1 eingreifen, um das Pumpengehäuse zusammenzuhalten.

An dem der Durchgangsöffnung 33 gegenüberliegenden Ende des Rahmens 30 ist eine Energiespeicheraufnahme 35 angeordnet. Sie ist entsprechend der Form eines Energiespeichers 6 geformt. Der Energiespeicher 6 ist in diesem Beispiel eine zylinderförmige, wieder aufladbare Batterie. Andere Formen und Arten von Energiespeichern lassen sich auch verwenden. Die Energiespeicheraufnahme 35 ist als ein in der Figur 1 nach oben offener Kasten ausgebildet, wobei auf zwei gegenüberliegenden Seiten je ein Klammerelement 34 nach oben ragt. All diese Elemente sind vorzugsweise einstückig gemeinsam mit dem Rahmen 30 ausgebildet.

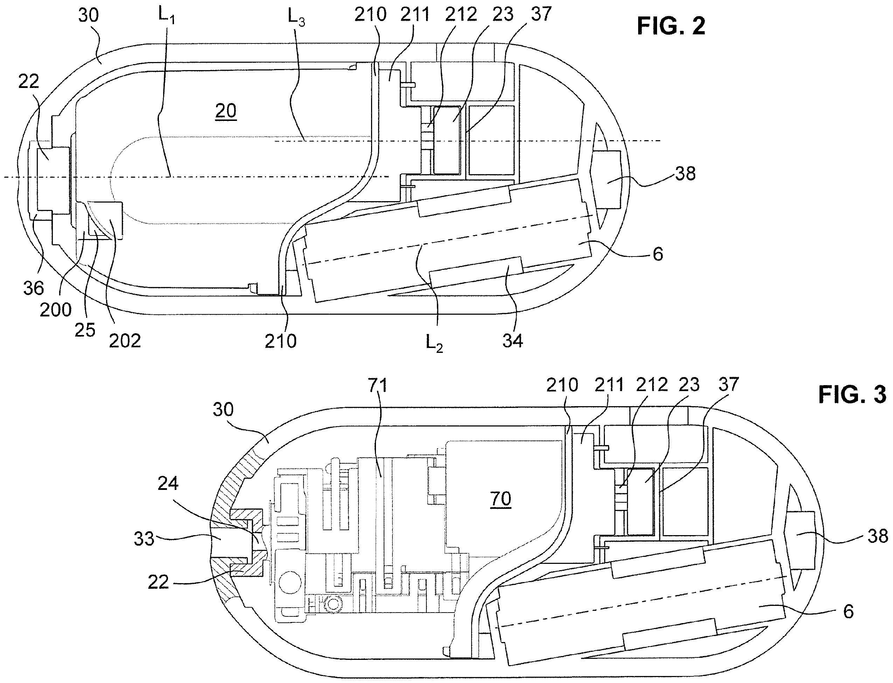

Benachbart zur Energiespeicheraufnahme 35 ist eine zweite Lageraufnahme 37 ausgebildet. Auch diese ist in diesem Beispiel als rechteckförmiger, nach oben offen ausgebildeter Kasten einstückig am Rahmen 30 angeformt. Das hintere, der Durchgangsöffnung 33 entgegengesetzte Ende des Rahmens 30 ist als Stromanschluss 38 ausgebildet. Dies ist in den Figuren 2 und 3 gut erkennbar.

Das Schalldämpfungsgehäuse 2 weist ein Etui 20 zur Aufnahme eines Pumpaggregats 7 auf. Das Etui 20 ist vorzugsweise steif oder halbsteif ausgebildet, insbesondere besteht es aus Kunststoff. Vorzugsweise weist es eine zur Pumpgehäuseform passende ovale abgerundete Form auf, wobei das Etui 20 im Bereich seiner Öffnung abgeschrägt ist. Die Abschrägung ist vorzugsweise geschwungen, so dass das Etui stufenförmig und auf einer Seite länger ausgebildet ist als auf der anderen Seite. Dies ist in Figur 1 gut erkennbar.

Dieser stufenförmige Rand ist mittels eines Deckels 21 geschlossen. Der Deckel 21 weist einen entsprechend geschwungen ausgebildeten, d.h. gebogenen Verschlusskörper 210 auf. Dieser geht vorzugsweise einstückig in einen annähernd zylinderförmigen Übergangsbereich 211 über. Der Durchmesser dieses Übergangsbereichs 211 entspricht vorzugsweise annähernd dem Durchmesser des längeren Bereichs des Etuis 20. Dem Übergangsbereich 211 folgt ein Verbindungsstift 212, an welchem sich ein zweites Lagermodul 23 anschliesst. Dies ist in den Figuren 2 und 3 gut erkennbar. Ein erstes Lagermodul 22 befindet sich am gegenüberliegenden Ende des Etuis 20 und wird weiter unten im Text ausführlicher beschrieben.

Das zweite Lagermodul 23 kann auf den Verbindungsstift 212 aufgesteckt sein. Vorzugsweise ist es jedoch einstückig mit diesem und dem gesamten restlichen Deckel 21 ausgebildet. Vorzugsweise ist der Deckel 21 mit dem zweiten Lager 23 elastisch ausgebildet. Insbesondere ist er weich. Vorzugsweise ist er aus einem Elastomer oder aus Silikon gefertigt.

Das Etui 20 weist im Bereich des ersten Lagermoduls 22 eine Ausnehmung 200 auf, welche im oberen Bereich ein Fenster 202 aufweist. In dieser Ausnehmung 200 ist ein Schalldämpfungselement 25, hier ein Schaumstoffwürfel, angeordnet.

In Figur 2 ist die Anordnung des Schalldämpfungsgehäuses 2 sowie des Energiespeichers 6 innerhalb des Rahmens 30 des Pumpaggregatträgers 3 gut erkennbar. Das Schalldämpfungsgehäuse 2, genauer das Etui 20, ist mittels des ersten Lagermoduls 22 in der ersten Lageraufnahme 36 gelagert. Das zweite Lagermodul 23 befindet sich in der zweiten Lageraufnahme 37 des Pumpaggregatträgers 3, so dass das Schalldämpfungsgehäuse 2 auch an diesem gegenüberliegenden Ende im Pumpaggregatträger 3 gelagert ist. Somit ist eine Zweipunktlagerung vorhanden.

Eine Durchgangsöffnung des ersten Lagermoduls 22, welche hier einen Vakuumanschluss 24 bildet, fluchtet mit der Durchgangsöffnung 33 des Pumpaggregatträgers 3, wie dies in Figur 3 gut erkennbar ist. Diese Durchgangsöffnung 33 bildet somit eine Aufnahme für einen Stecker eines Saugschlauchs, welcher zu einer Brusthaube zum Anlegen an die Mutterbrust führt. Stecker, Saugschlauch und Brusthaube sind hier nicht dargestellt. Sie sind jedoch aus dem Stand der Technik hinlänglich bekannt.

Wie in Figur 2 erkennbar ist, definiert das Etui 20 eine erste Längsachse L₁, welche mit der Längsmittelachse des Vakuumanschlusses 24 fluchtet. Der Energiespeicher 6 definiert einen zweite Längsachse L₂, welche in einem Winkel zu dieser ersten Längsachse L₁ verläuft. Das zweite Lagermodul 23 definiert mit seiner Längsmittelachse eine dritte Längsachse L₃, welche parallel versetzt zur ersten Längsachse L₁ verläuft.

Wie in den Figuren 2 und 3 gut erkennbar ist, ist der Platz innerhalb des Pumpaggregatträgers durch die Abschrägung des Etuis 20 sowie durch die schräge Anordnung des Energiespeichers 6 optimal genutzt und Leerräume sind weitgehend vermieden. Das Pumpengehäuse kann insgesamt sehr klein und kompakt ausgebildet werden. Es ist schallgedämpft und trotzdem steif und robust.

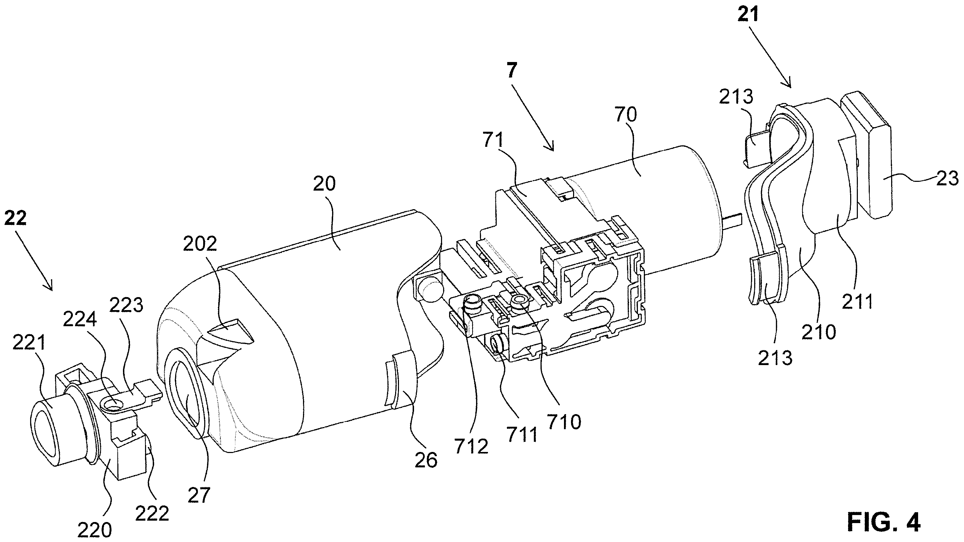

Die Anordnung des Pumpaggregats 7 innerhalb des Schalldämpfungsgehäuses 2 ist in Figur 3 dargestellt. Eine passende Explosionsdarstellung dazu findet sich in Figur 4.

Vorzugsweise entspricht das Pumpaggregat 7 im Wesentlichen dem in WO 2006/032156 A1 beschriebenen Pumpaggregat. Es lassen sich jedoch auch andere Formen und Ausgestaltungen in der erfindungsgemässen Vorrichtung verwenden. Vorzugsweise umfasst das Pumpaggregat jedoch einen Elektromotor sowie eine Pumpeinheit, insbesondere eine Membranpumpe, vorzugsweise eine Pumpkammer mit einer Pumpmembran.

Der Elektromotor ist in den Figuren mit dem Bezugszeichen 70 versehen, die Pumpeinheit mit dem Bezugszeichen 71. Die dritte Längsachse L₃ des zweiten Lagermoduls 23 bildet die Flucht einer nicht dargestellten Motorachse des Elektromotors 70. Der Elektromotor 70 ist somit benachbart zum zweiten Lagermodul 23 angeordnet.

Die Pumpeinheit 71 weist eine Entlüftungsöffnung 710, eine Vakuumöffnung 711 sowie eine Belüftungsöffnung 712 auf. Dies ist in Figur 3 gut erkennbar.

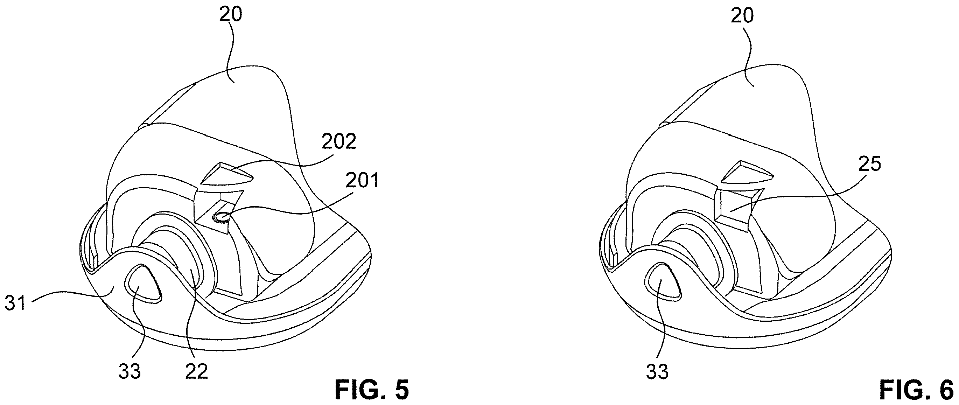

Die Belüftungsöffnung 712 schliesst sich im zusammengebauten Zustand der Vorrichtung an eine Lufteinlassöffnung 201 an, welche sich in der Ausnehmung des Schalldämpfungsgehäuses 2 befindet. Diese Lufteinlassöffnung 201 ist in Figur 5 gut erkennbar. Sie ist vom Schalldämpfungselement 25 überdeckt, wie dies in Figur 6 dargestellt ist. Ein Fenster 202 ist aus spritzgusstechnischen Gründen im Etui 20 vorhanden.

Die Entlüftungsöffnung 710 führt in das Innere des Schalldämpfungsgehäuses 2, genauer des Etuis 20. Luft, welche über diese Entlüftungsöffnung 710 aus der Pumpeinheit 71 entweicht, verlässt das Schalldämpfungsgehäuse 2 über undichte Stellen wie beispielsweise über ein Aufnahmeelement 26 einer Steckverbindung mit einem Einsteckelement 213 des Deckels 21.

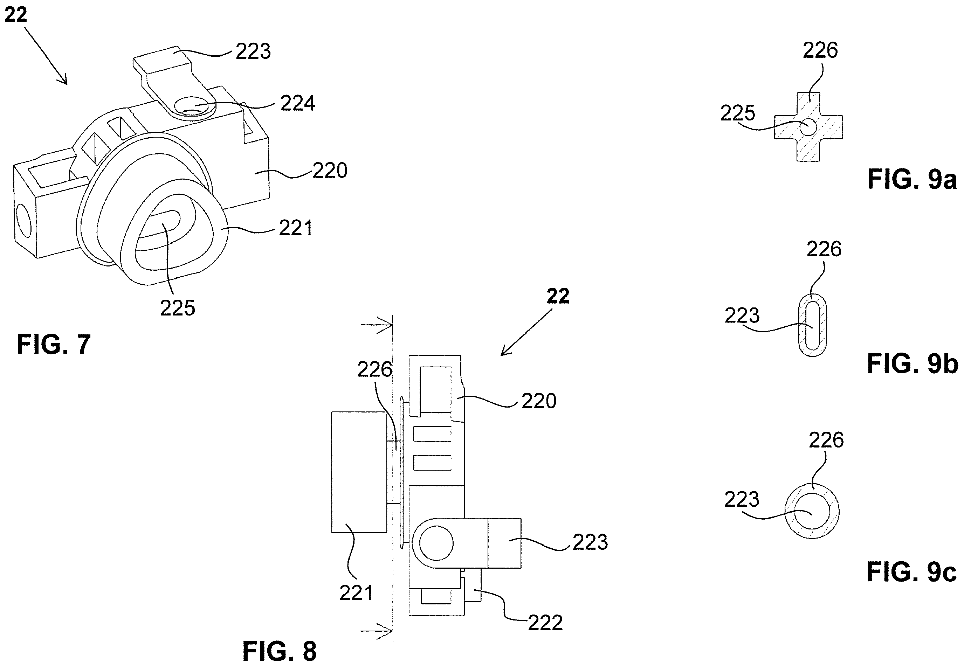

Die Vakuumöffnung 711 fluchtet mit einem Verbindungskanal 222 des ersten Lagermoduls 22, wie dies in Figur 4 erkennbar ist. Der Verbindungskanal 222 weist im Innern des ersten Lagermoduls 22 einen abgewinkelten, d.h. nicht geradlinigen Verlauf auf und endet im Vakuumanschluss 24, genauer in der darin vorhandenen Anschlussöffnung 225. Diese Anschlussöffnung 225 ist in Figur 7 gut erkennbar.

Das Pumpaggregat 7 ist mittels des ersten Lagermoduls 22 vakuumseitig im Schalldämpfungsgehäuse 2 gelagert und mittels des zweiten Lagermoduls 23, genauer des Deckels 21, motorseitig im Schalldämpfungsgehäuse 2 gelagert. Der Deckel 21 ist hierfür lediglich auf dem Etui 20 aufzustecken und mit den bereits erwähnten Einsteckelementen 213 in die entsprechenden Aufnahmeelemente 26 einzustecken.

Das erste Lagermodul 22 ist nicht wie in Figur 4 dargestellt dem Etui 20 vorgelagert, sondern es befindet sich innerhalb des Etuis 20. Es lässt sich hierfür einfach über die gebogene Öffnung einschieben und durch eine stirnseitige Durchgangsöffnung 27 des Etuis 20 teilweise durchschieben. Ein Anschlusskörper 221 ragt aus der Öffnung heraus, ein Hals 226 ist freigestellt und ein Grundkörper 220 dichtet gegenüber dem Innenraum des Etuis 20 an dessen Innenseite. Diese Teile sind in den Figuren 7 und 8 gut erkennbar. Zur Befestigung des Grundkörpers 220 im Etui 20 dient vorzugsweise ein Klemmelement 223 in der Form einer federnden Zunge. Diese Zunge 223 dichtet ebenfalls gegenüber dem Innenraum des Etuis 20.

Der Grundkörper 220 weist einen Belüftungskanal 224 auf, welcher die Verbindung zwischen der Belüftungsöffnung 712 der Pumpeinheit 71 und der Lufteinlassöffnung 201 des Etuis 20 schafft.

Das gesamte erste Lagermodul 22 ist einstückig und vorzugsweise elastisch, insbesondere weich ausgebildet. Es bildet somit an zwei gegenüberliegenden Seiten Lagerstellen aus: auf einer ersten Seite zwischen dem Schalldämpfungsgehäuse 2 und dem Pumpaggregatträger 3 und somit dem Pumpengehäuse und auf einer gegenüberliegenden Seite zwischen dem Schalldämpfungsgehäuse 2 und dem Pumpaggregat 7, genauer der Pumpeinheit 71.

Auch der Deckel mit dem zweiten Lagermodul 23 ist einstückig und vorzugsweise elastisch, insbesondere weich ausgebildet. Auch dieses bildet somit an zwei gegenüberliegenden Seiten Lagerstellen aus: auf einer ersten Seite zwischen dem Schalldämpfungsgehäuse 2 und dem Pumpaggregatträger 3 und somit dem Pumpengehäuse und auf einer gegenüberliegenden Seite zwischen dem Schalldämpfungsgehäuse 2 und dem Pumpaggregat 7, genauer dem Elektromotor 70. Da der Pumpaggregatträger 3 Teil des äusseren Pumpengehäuses ist, erübrigt sich eine weitere Lagerung.

Die Schalldämpfung bzw. die Vibrationsdämpfung lässt sich weiterhin optimieren, indem die Anschlussöffnung 225 entsprechend gewählt wird. Bewährt haben sich Formen, wie sie in den Figuren 9a bis 9c dargestellt sind. Es ist der Hals 226 des ersten Lagermoduls 22 im Querschnitt gezeigt sowie die sich darin befindliche Anschlussöffnung 225. Die Querschnitte des Halses 226 sowie die Formen der Anschlussöffnung 225 lassen sich beliebig miteinander kombinieren. In Figur 9a ist der Hals 226 kreuzförmig und die Anschlussöffnung 225 ist rund. In Figur 9b sind der Hals 226 und die Anschlussöffnung 225 oval und in Figur 9c sind sie rund. Die Kombination gemäss Figur 9b ist am meisten bevorzugt.

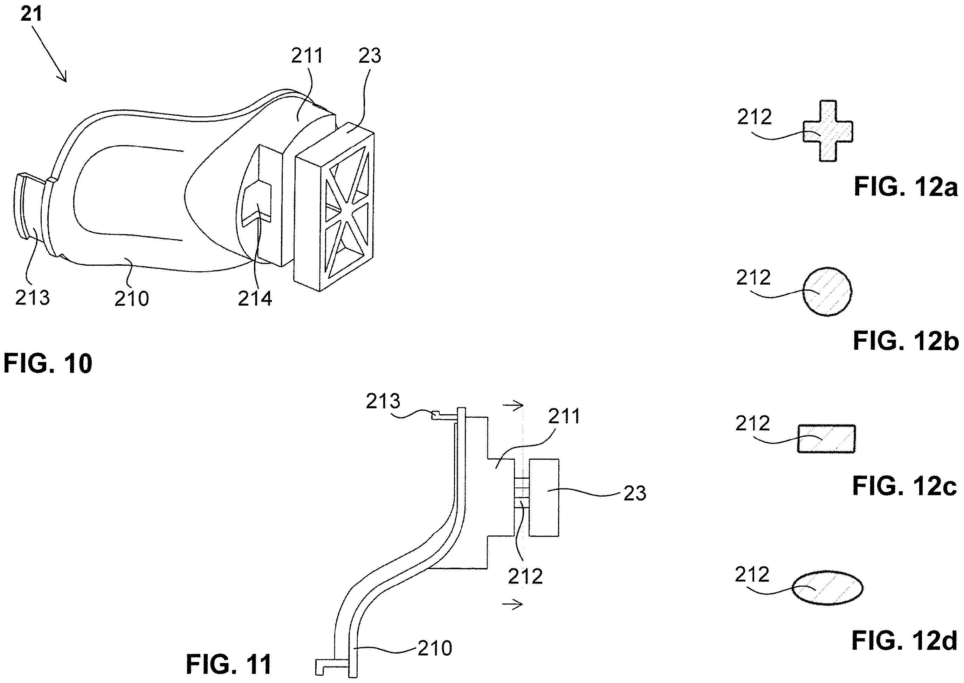

In den Figuren 10 bis 12d ist der Deckel 21 mit dem zweiten Lagermodul 23 dargestellt. Er weist wie bereits beschrieben den gebogenen Verschlusskörper 210, den Übergangsbereich 211, den Verbindungsstift 212 und das zweite Lagermodul 23 auf. Das zweite Lagermodul 23 weist vorzugsweise einen quaderförmigen Rahmen mit darin verlaufenden Verbindungsstreben auf. Dadurch ist er elastisch und passt sich seiner zugehörigen zweiten Lageraufnahme 37 optimal an.

Im Übergangsbereich 211 sind vorzugsweise Kabeldurchführungen 214 in Form von Durchgangsöffnungen vorhanden, um den Elektromotor 70 über die Grundplatte 40 mit der Steuer- und Elektronikeinheit 41 zu verbinden. Am gebogenen Verschlusskörper 210 sind die Einsteckelemente 213 angeformt.

Die Schalldämpfung und die Lagerung lassen sich durch die Formgebung des Verbindungsstifts 212 zusätzlich optimieren. In den Figuren 12a bis 12 sind bevorzugte Varianten dargestellt. Vorzugsweise ist der Verbindungsstift 212 vollflächig, also massiv ausgebildet. Vorzugsweise ist er relativ steif.

In Figur 12a ist sein Querschnitt kreuzförmig, in Figur 12b rund, in Figur 12c rechteckig und in Figur 12d oval. Vorzugsweise ist er kreuzförmig.

Die erfindungsgemässe Saugpumpe ist klein und kompakt und weist eine gute Schalldämpfung auf.

### BEZUGSZEICHENLISTE

| | | | |
|---|---|---|---|
| 1 | erstes Pumpengehäuseteil | 30 | Rahmen |
| 10 | schalenförmiger Grundkörper | 31 | Stirnwand |
| 11 | Auflagefläche | 32 | drittes Verbindungselement |
| 12 | erstes Verbindungselement | 33 | Durchgangsöffnung |
| | | 34 | Klammerelement |
| 2 | Schalldämpfungsgehäuse | 35 | Energiespeicheraufnahme |
| 20 | Etui | 36 | erste Lageraufnahme |
| 200 | Ausnehmung | 37 | zweite Lageraufnahme |
| 201 | Lufteinlassöffnung | 38 | Stromanschluss |
| 202 | Fenster | | |
| 21 | Deckel | 4 | zweites Pumpengehäuseteil |
| 210 | gebogener Verschlusskörper | 40 | Grundplatte |
| 211 | Übergangsbereich | 41 | Steuer- und Elektronikeinheit |
| 212 | Verbindungsstift | 42 | zweites Verbindungselement |
| 213 | Einsteckelement | 43 | Rinne |
| 214 | Kabeldurchführungen | | |
| 22 | erstes Lagermodul | 5 | Abdeckung |
| 220 | Grundkörper | 50 | Deckplatte |
| 221 | Anschlusskörper | 51 | Anzeige- und Betätigungsfenster |
| 222 | Verbindungskanal | | |
| 223 | Klemmelement | | |
| 224 | Belüftungskanal | 6 | Energiespeicher |
| 225 | Anschlussöffnung | | |
| 226 | Hals | 7 | Pumpaggregat |
| 23 | zweites Lagermodul | 70 | Elektromotor |
| 24 | Vakuumanschluss | 71 | Pumpeinheit |
| 25 | Schalldämpfungselement | 710 | Entlüftungsöffnung |
| 26 | Aufnahmeelement | 711 | Vakuumöffnung |
| 27 | Durchgangsöffnung | 712 | Belüftungsöffnung |
| | | | |
| 3 | Pumpaggregatträger | L₁ | erste Längsachse |
| L₂ | zweite Längsachse | | |
| L₃ | dritte Längsachse | | |

## Patentansprüche

1. Motorbetriebene medizinische Saugpumpe mit einem Pumpaggregat zur Erzeugung eines Unterdrucks, mit einem Pumpaggregatträger (3) zur Halterung des Pumpaggregats (7) und mit einer Aufnahme (35) zur Aufnahme eines Energiespeichers (7), wobei das im Pumpaggregatträger (3) angeordnete Pumpaggregat (7) eine erste Längsachse (L₁) definiert und die Aufnahme (35) eine zweite Längsachse (L₂) definiert, **dadurch gekennzeichnet, dass** der Pumpaggregatträger (3) die Aufnahme (35) bildet und dass die zweite Längsachse (L₂) in einem Winkel zur ersten Längsachse (L₁) verläuft.

2. Motorbetriebene medizinische Saugpumpe nach Anspruch 1, wobei der Pumpaggregatträger (3) im Wesentlichen als Rahmen (30) ausgebildet ist und eine im Wesentlichen längliche abgerundete Grundform, insbesondere einen elliptische oder ovale Grundform, aufweist.

3. Motorbetriebene medizinische Saugpumpe nach einem der Ansprüche 1 oder 2, wobei sie ein Pumpengehäuse mit einem ersten Pumpengehäuseteil (1) und einem zweiten Pumpengehäuseteil (4) umfasst und wobei der Pumpaggregatträger (3) zwischen dem ersten und dem zweiten Pumpengehäuseteil (1, 4) gehalten ist.

4. Motorbetriebene medizinische Saugpumpe nach einem der Ansprüche 1 bis 3, wobei eine Lufteinlassöffnung (201) vorhanden ist, welche von einem Schalldämpfungselement (25) überdeckt ist.

5. Motorbetriebene medizinische Saugpumpe nach einem der Ansprüche 1 bis 4, wobei mindestens zwei elastische Lager (22, 23) vorhanden sind zur Lagerung des Pumpaggregats (7) im Pumpaggregatträger (3).

6. Motorbetriebene medizinische Saugpumpe nach Anspruch 5, wobei mindestens ein erstes dieser Lager (22) einen Vakuumanschluss (24) bildet und mindestens ein zweites dieser Lager (23) an einem motorseitigen Ende des Pumpaggregats (7) und in Verlängerung einer Motorachse des Pumpaggregats (7) angeordnet ist.

7. Motorbetriebene medizinische Saugpumpe nach einem der Ansprüche 5 oder 6, wobei genau zwei Lager (22, 23) vorhanden sind, welche entlang der ersten Längsachse (L₁) an gegenüberliegenden Enden des Pumpaggregats angeordnet sind.

8. Motorbetriebene medizinische Saugpumpe nach Anspruch 7, wobei die zwei Lager (22, 23) bezüglich der ersten Längsachse (L₁) versetzt zueinander angeordnet sind.

9. Motorbetriebene medizinische Saugpumpe nach einem der Ansprüche 1 bis 8, wobei das Pumpaggregat (7) in einem Schalldämpfungsgehäuse (2) angeordnet ist und das Schalldämpfungsgehäuse (2) im Pumpaggregatträger (3) gehalten ist.

10. Motorbetriebene medizinische Saugpumpe nach Anspruch 9, wobei das Schalldämpfungsgehäuse (2) ein Etui (20) mit einer Aufnahmeöffnung zur Aufnahme des Pumpaggregats (7) und einen das Etui (20) verschliessenden Deckel (21) aufweist, wobei der Deckel (21) gebogen ausgebildet ist.

11. Motorbetriebene medizinische Saugpumpe nach den Ansprüchen 5 und 9, wobei das Schalldämpfungsgehäuse (2) mittels der mindestens zwei elastischen Lager (22, 23) im Pumpaggregatträger (3) gelagert ist.

12. Motorbetriebene medizinische Saugpumpe nach Anspruch 11, wobei die mindestens zwei elastischen Lager (22, 23) einerseits das Schalldämpfungsgehäuse (2) im Pumpengehäuse (1, 3, 4) lagern und andererseits das Pumpaggregat (7) innerhalb des Schalldämpfungsgehäuses (2) lagern.

13. Motorbetriebene medizinische Saugpumpe nach Anspruch 12, wobei das erste Lager (22) ein elastisches Einsatzelement ist, welches im Schalldämpfungsgehäuse (2) gehalten ist und dieses durchsetzt.

14. Motorbetriebene medizinische Saugpumpe nach einem der Ansprüche 12 oder 13, wobei das zweite Lager (23) Teil eines Deckels (21) des Schalldämpfungsgehäuses (2) ist, wobei der gesamte Deckel (21) elastisch ausgebildet ist.

15. Medizinische Saugpumpe mit einem Pumpengehäuse (1, 3, 4), mit einem im Pumpengehäuse (1, 3, 4) angeordneten Pumpaggregat (7) zur Erzeugung eines Unterdrucks und mit mindestens einem ersten elastischen Lager (22) und einem zweiten elastischen Lager (23) zur Lagerung des Pumpaggregats (7) im Pumpengehäuse (1, 3, 4), wobei das erste elastische Lager (22) sich an einem ersten Ende des Pumpaggregats (7) befindet und das zweite elastische Lager (23) sich an einem zweiten, dem ersten Ende gegenüberliegenden Ende des Pumpaggregats (7) befindet, **dadurch gekennzeichnet, dass** das erste elastische Lager (22) einen Vakuumanschluss (24) bildet.

16. Medizinische Saugpumpe mit einem Pumpaggregat (7), einem Schalldämpfungsgehäuse (2) zur Aufnahme des Pumpaggregats (7) und mit einem Pumpengehäuse (1, 3, 4) zur Aufnahme des Schalldämpfungsgehäuses (2), **dadurch gekennzeichnet, dass** das Schalldämpfungsgehäuse (2) ein Etui (20) zur Aufnahme des Pumpaggregats (7) und einen das Etui (20) verschliessenden Deckel (21) aufweist, wobei der Deckel (21) elastisch ausgebildet ist und ein elastisches Lager (23) zur Lagerung des Schalldämpfungsgehäuses (2) im Pumpengehäuse (1, 3, 4) aufweist.

17. Medizinische Saugpumpe mit einem Pumpaggregat (7), einem Schalldämpfungsgehäuse (2) zur Aufnahme des Pumpaggregats (7) und mit einem Pumpengehäuse (1, 3, 4) zur Aufnahme des Schalldämpfungsgehäuses (2), **dadurch gekennzeichnet, dass** mindestens ein erstes und ein zweites elastisches Lager (22, 23) vorhanden sind, wobei mindestens eines dieser zwei elastischen Lager (22, 23) einerseits das Schalldämpfungsgehäuse (2) im Pumpengehäuse (1, 3, 4) lagert und andererseits das Pumpaggregat (7) innerhalb des Schalldämpfungsgehäuses (2) lagert.
